# EUROPEAN PATENT SPECIFICATION

(11) **EP 4 412 681 B1**
(45) Date of publication and mention of the grant of the patent: **09.12.2026**
(21) Application number: 22797975.4
(22) Date of filing: 05.10.2022
(51) Int. Cl.: A61M 5/20, A61M 5/315, A61M 5/32, A61M 5/24

(54) **IMPACT ACTIVATED RETENTION FEATURE FOR DRUG DELIVERY DEVICE**
STOSSAKTIVIERTE RÜCKHALTEFUNKTION FÜR EINE ARZNEIMITTELABGABEVORRICHTUNG
ÉLÉMENT DE RETENUE ACTIVÉ PAR IMPACT POUR DISPOSITIF D'ADMINISTRATION DE MÉDICAMENT

(30) Priority: 06.10.2021 US 202163252940 P
(43) Date of publication of application: 14.08.2024
(73) Proprietor: AMGEN INC., Thousand Oaks, CA 91320-1799 (US)
(72) Inventor: SPORK, Emil Gram, Thousand Oaks, CA 91320-1799 (US); MELANDER, Matias, Thousand Oaks, CA 91320-1799 (US); GEGELASHVILI, Alexandre, Thousand Oaks, CA 91320-1799 (US); SONG, Yangkun, Thousand Oaks, CA 91320-1799 (US); SONDERBY, Thomas Peter, Thousand Oaks, CA 91320-1799 (US)
(74) Representative: J A Kemp LLP
(86) International application number: PCT/US2022/045716
(87) International publication number: WO 2023/059671

(56) References cited:
- WO-A1-2018/011417
- CA-A1- 3 110 529
- US-A1- 2021 260 302

## Description

### FIELD OF DISCLOSURE

The present disclosure generally relates to drug delivery devices, and, more particularly, to impact activated retention features for drug delivery devices.

### BACKGROUND

Drug delivery devices, such as injectors, are used to deliver liquid drugs to a patient. Upon activation, a drug delivery device will expel a drug stored within an internal reservoir through a needle, cannula, or other delivery member into the patient. Some drug delivery devices, such as pen-type autoinjectors, may be positioned adjacent to a patient's skin to deliver a drug via an injection needle or some other means over a period of time. The drug delivery device may be positioned near the tissue of the patient's abdomen, thigh, arm, or some other portion of the patient's body. US 2021/260302 A1 discloses an activation mechanism for a drug delivery device. WO 2018/011417 A1 discloses a medical injector having a safety feature. CA 3110529 A1 discloses injection systems for drug delivery devices.

Some devices may have drawbacks. Specifically, users may be frightened by an exposed injection needle or feel they are inherently incapable of performing an injection. Because of aversions to exposed needles, as well as health and safety issues that may be involved, various types of injectors and other devices have been developed for concealing needles from the user and automating the injection task to assist the user in performing the injection, ensure reliable delivery of the medication and ensure patient safety.

Typically, three tasks may be performed when injecting a drug into a patient with a hypodermic syringe: 1) insertion of the needle into the patient; 2) injection of the drug from the syringe into the patient; and 3) withdrawal of the needle after the injection has been completed. Generally, shield-activated devices use manual needle insertion techniques whereby a user simultaneously inserts a needle and initiates dosing through the action of retracting a shield relative to the rest of the device. In these devices, the needle may automatically insert upon manually activating the device. Button-activated devices typically employ automated needle insertion mechanisms whereby the needle is inserted mechanically and the dosing mechanism release is automatically delayed until the correct device state is achieved. Any or all of these devices may use manual and/or automated withdrawal mechanisms to retract the needle, and typically rely on springs or other power sources to generate forces required to perform the tasks. Occasionally, a user may inadvertently mishandle or drop the device prior to use. In these circumstances, if the device is dropped in certain orientations, inertial forces may cause the internal components to move relative to each other, which may result in inadvertent premature device activation. Such a premature activation may result in some or all of the desired drug from actually being delivered to the user, which may be wasteful and potentially harmful to the user and/or others.

The present disclosure sets forth drug delivery devices embodying advantageous alternatives to existing drug delivery devices, and that may address one or more of the challenges or needs mentioned herein.

### SUMMARY

The invention is defined in the appended claims. In accordance with a first aspect, a drug delivery device includes a housing having proximal and distal ends and a longitudinal axis extending therebetween, an injection assembly at least partially disposed within the housing and including a needle or a cannula, a drive assembly operably coupled with the injection assembly, a shield slidably coupled with the housing and operably coupled with the drive assembly, and a retention mechanism. The drive assembly is engageable to deliver a medicament via the injection assembly. The shield is positionable in an extended position in which at least a proximal end extends a distance beyond the proximal end of the housing and a retracted position where the proximal end of the housing protrudes a distance beyond the proximal end of the shield. Moving the shield to the retracted position engages the drive assembly to deliver the medicament via the injection assembly. The retention mechanism limits movement of the drive assembly to restrict engagement thereof such that the drive assembly is restricted from delivering the medicament via the injection assembly during unintentional movement of the housing.

In some examples, the drive assembly may further include a trigger ring engageable by the shield. The trigger ring may be movable between an initial position and a releasing position. In some of these approaches, movement of the shield to the retracted position urges the trigger ring to the releasing position. In these and other examples, the shield may include an activator portion that engages the trigger ring.

In some approaches, the shield may include an activator portion that engages the trigger ring. Further, the retention mechanism may include at least one arm that is carried by a nut. The at least one arm may engage a portion of the trigger ring to prevent the trigger ring from moving to the releasing position. In some examples, the device may further include a container holder operably coupled with the injection assembly. The container holder may include an arm that engages the at least one arm during inadvertent or unintended movement of the device. In some examples, the container holder may be fixedly coupled with the housing.

In accordance with a second aspect, a drug delivery device may include a housing having a proximal end, a distal end, and a longitudinal axis extending between the proximal end and the distal end thereof, an injection assembly at least partially disposed within the housing at or near the proximal end, a drive assembly at least partially disposed within the housing and operably coupled with the injection assembly, and a shield slidably coupled with the housing and operably coupled with the drive assembly. The drive assembly may include a trigger ring being movable between an initial position and a releasing position to deliver a medicament via the injection assembly, and further may include a nut at least partially disposed around a periphery of the trigger ring. The shield is positionable in an extended position in which at least a proximal end of the shield extends a distance beyond the proximal end of the housing and a retracted position in which the proximal end of the housing protrudes a distance beyond the proximal end of the shield. Upon moving the shield to the retracted position, a portion of the shield urges the trigger ring to the releasing position to deliver the medicament via the injection assembly. The container holder, the nut, and the trigger ring cooperate to form a retention mechanism to prevent the trigger ring from activating to deliver the medicament via the injection assembly

### BRIEF DESCRIPTION OF THE DRAWINGS

The above needs are at least partially met through provision of the impact activated retention feature for drug delivery devices described in the following detailed description, particularly when studied in conjunction with the drawings, wherein:

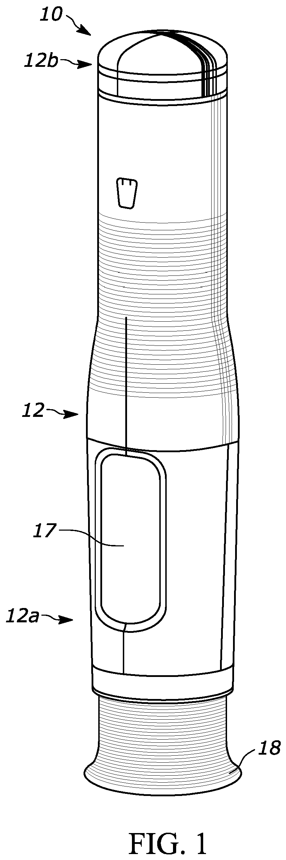

Fig. 1 illustrates a perspective view of an example drug delivery device in accordance with various embodiments;
Fig. 2 illustrates a cross-sectional view of the example drug delivery device of Fig. 1 in accordance with various embodiments;
Fig. 3A illustrates a cross-sectional view of an example rear sub-assembly of the example drug delivery device of Figs. 1 & 2 in accordance with various embodiments;
Fig. 3B illustrates a cross-sectional view of an example drug storage container of the example drug delivery device of Figs. 1-3A in accordance with various embodiments;
Fig. 3C illustrates a cross-sectional view of an example front sub-assembly of the example drug delivery device of Figs. 1-3B in accordance with various embodiments;
Fig. 4 illustrates a perspective view of an example drive assembly of the example drug delivery device of Figs. 1-3C in accordance with various embodiments;
Fig. 5 illustrates a cross-sectional view of the example drive assembly of the example drug delivery device of Figs. 1-4 in a pre-activated state and prior to impact in accordance with various embodiments;
Fig. 6 illustrates a cross-sectional view of the example drive assembly of the example drug delivery device of Figs. 1-5 in a pre-activated state and during impact in accordance with various embodiments; and
Fig. 7 illustrates a cross-sectional view of the example drive assembly of the example drug delivery device of Figs. 1-6 in a pre-activated state and after impact in accordance with various embodiments.

Skilled artisans will appreciate that elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present invention. Also, common but well-understood elements that are useful or necessary in a commercially feasible embodiment are often not depicted in order to facilitate a less obstructed view of these various embodiments. It will further be appreciated that certain actions and/or steps may be described or depicted in a particular order of occurrence while those skilled in the art will understand that such specificity with respect to sequence is not actually required. It will also be understood that the terms and expressions used herein have the ordinary technical meaning as is accorded to such terms and expressions by persons skilled in the technical field as set forth above except where different specific meanings have otherwise been set forth herein.

### DETAILED DESCRIPTION

Generally speaking, pursuant to these various embodiments, a drug delivery device is provided that prevents premature activation of the device both during and after an inadvertent drop. The drug delivery device provided herein incorporates a retention mechanism that retains components used to activate the device in instances where drop-related forces may occur. When a falling device comes to a stop due to contact with a surface (e.g., the floor, a table, etc.), the internal components ordinarily would move due to these inertial forces. However, the components of the device that are responsible for device activation are prevented from traveling to the same extent, and by using this relative difference in movement, the retention mechanism may remove kinetic energy from the device. After the impact, the internal components will return to their default location and the device will function as intended.

Turning to the Figures, a drug delivery device 10 for delivering a drug, which may also be referred to herein as a medicament or drug product, is provided. The drug may be, but is not limited to, various biologicals such as peptides, peptibodies, or antibodies. The drug may be in a fluid or liquid form, although the disclosure is not limited to a particular state. In certain liquid formulations, the drug may have a viscosity between approximately (e.g., ±10%) 1 - 13 centipoise (cP), approximately (e.g., ±10%) 1 - 30cP, approximately (e.g., ±10%) 1 - 60cP, or other suitable viscosity profiles. Other examples are possible.

Various implementations and configurations of the drug delivery device 10 are possible. For example, the present disclosure describes a drug delivery device 10 is in the form of a single-use, disposable injector. In other embodiments, the drug delivery device 10 may be configured as multiple-use reusable injector. The drug delivery device 10 is operable for selfadministration by a patient or for administration by caregiver or a formally trained healthcare provider (e.g., a doctor or nurse). Further, in the illustrated examples, the drug delivery device 10 takes the form of an autoinjector or pen-type injector, and, as such, may be held in the hand of the user over the duration of drug delivery or dosing.

The configuration of various components included in the drug delivery device 10 may depend on the operational state of the drug delivery device 10. The drug delivery device 10 may have a pre-delivery or storage state, a delivery or dosing state, and a post-delivery state, although fewer or more states are possible. The pre-delivery state may correspond to the configuration of the drug delivery device 10 subsequent to assembly and prior to activation by the user. In some embodiments, the pre-delivery state may exist in the time between when the drug delivery device 10 leaves a manufacturing facility and when a patient or user activates a drive assembly of the drug delivery device 10. The delivery state may correspond to the configuration of the drug delivery device 10 while drug delivery is in progress. It is appreciated that during a transition from the pre-delivery state and the delivery state, the user may remove the drug delivery device 10 from any secondary packaging and begin positioning the drug delivery device 10 against the injection site. The post-delivery state may correspond to the configuration of the drug delivery device 10 after drug delivery is complete and/or when a stopper is arranged in an end-of-dose position in a drug storage container. For the purposes of the present disclosure, only the pre-delivery state and a portion of the delivery state will be described herein, as the braking mechanism described herein serves to maintain the drug delivery device 10 in the pre-delivery state in the event of accidental and/or unintended drops or contact.

The drug delivery device 10 includes an outer casing or housing 12. In some embodiments, the housing 12 may be sized and dimensioned to enable a person to grasp the injector 10 in a single hand. The housing 12 may have a generally elongate shape, such as a cylindrical shape, and extend along a longitudinal axis A between a proximal end 12a and a distal end 12b. The drug delivery device 10 further includes an injection assembly 15 and a drive assembly 30. The injection assembly 15 and the drive assembly 30 may each be at least partially disposed within the housing 12. The injection assembly 15 includes a delivery member 16 in the form of a needle or a cannula. An opening 14 may be formed in the proximal end 12a to permit an insertion end 16a of the delivery member 16 to extend outside of (i.e., beyond the length of) the housing 12.

A transparent or semi-transparent inspection window 17 may be positioned in a wall of the housing 12 to permit a user to view component(s) inside the drug delivery device 10, including a drug storage container 23 (which is also part of the injection assembly 15). Viewing the drug storage container 23 through the window 17 may allow a user to confirm that drug delivery is in progress and/or complete. A removable cap 18 may cover the opening 14 prior to use of the drug delivery device 10, and, in some embodiments, may including a gripper 21a configured to assist with removing a sterile barrier 21 (e.g., a rigid needle shield (RNS), a flexible needle shield (FNS), etc.) mounted on the insertion end 16a of the delivery member 16. The gripper 21a may include one or more inwardly protruding barbs or arms that frictionally or otherwise mechanically engage the sterile barrier 21 to pull the sterile barrier 21 with the removable cap 18 when the user separates the removable cap 18 from the housing 12. Thus, removing the removable cap 18 has the effect of removing the sterile barrier 21 from the delivery member 16.

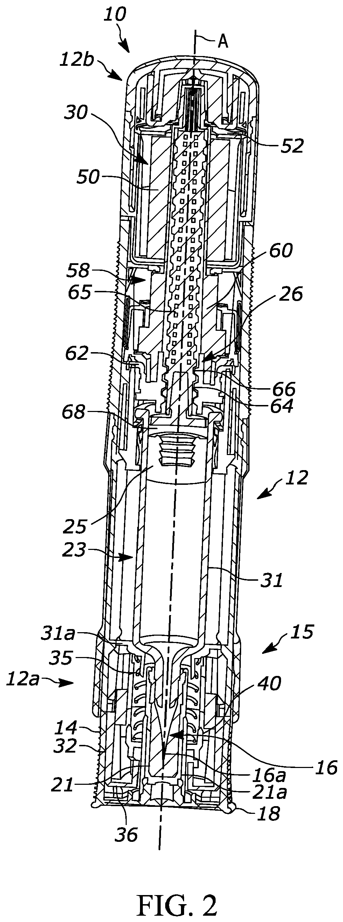

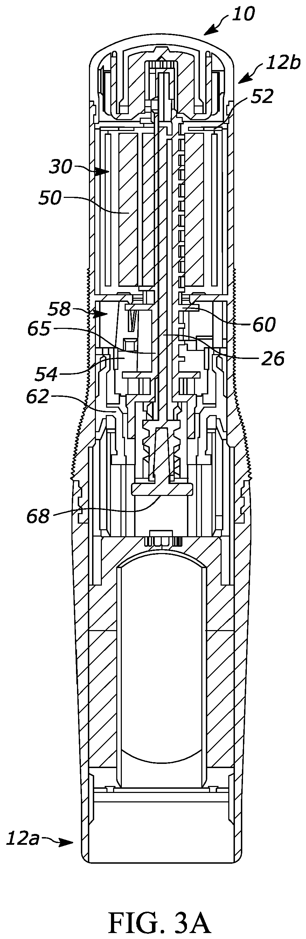

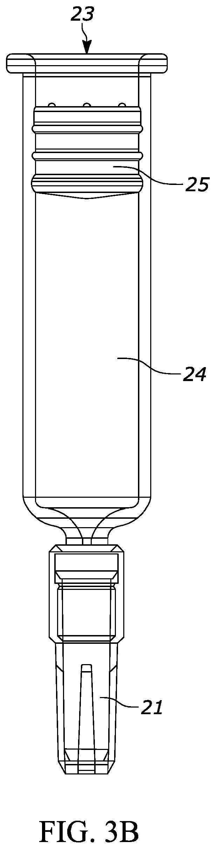

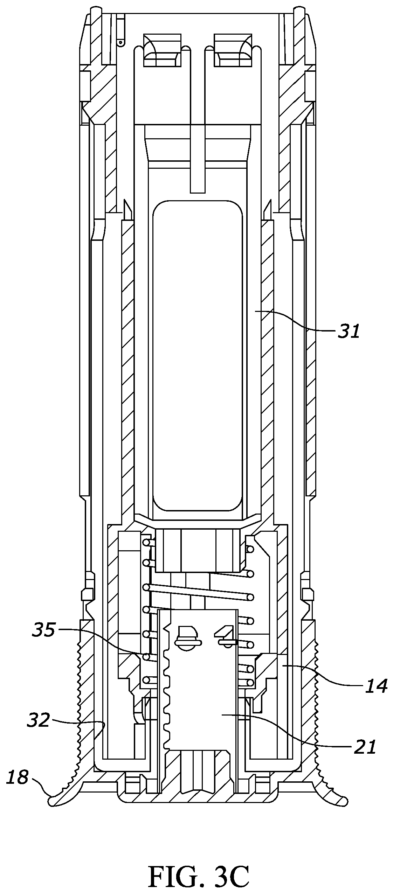

The cap 18 is in the form of a generally hollow member that may be removably coupled with the housing 12 and/or a shield 32. More specifically, in the illustrated example of Figs. 2 and 3C, a portion of the cap 18 is insertable into the opening 14 formed by the housing 12.

The housing 12 may have a hollow and generally cylindrical or tubular shape, and may include a rear cover having a generally hemispherical shape or a hollow cylindrical shape with an open end and a closed off end. In some embodiments, the housing, and any components to be contained therein, may be assembled together to define various sub-assemblies (e.g., a rear sub-assembly as illustrated in Fig. 2A and a front sub-assembly as illustrated in Fig. 2C). In some embodiments, the rear and front sub-assemblies are assembled independently of each other and then later combined with one another, as well as with the drug storage container 23, to form the fully-assembled drug delivery device 10. In certain such embodiments, some or all of the foregoing phases of assembly may occur in different manufacturing facilities or environments. In alternative embodiments, the housing 12 may be constructed in one piece, such that the housing 12 is defined by single, monolithic structure.

The drug storage container 23 is disposed within an interior space of the housing 12 and is configured to contain a drug 24. The drug storage container 23 may be pre-filled and shipped, e.g., by a manufacturer, to a location where the drug storage container 23 is combined with a remainder of the drug delivery device 10. The housing 12 may be pre-loaded with the drug storage container 23, e.g., by a manufacturer, or alternatively, loaded with the drug storage container 23 by a user prior to use of the drug delivery device 10. The drug storage container 23 may include a rigid wall defining an internal bore or reservoir. The wall may be made of glass or plastic. A stopper 25 may be moveably disposed in the drug storage container 23 such that it can move in an axial direction along the longitudinal axis A between the distal end and the proximal end the drug storage container 23. The stopper 25 may be constructed of rubber or any other suitable material. The stopper 25 may slidably and sealingly contact an interior surface of the wall of the drug storage container 23 such that the drug 24 is prevented or inhibited from leaking past the stopper 25 when the stopper 25 is in motion. Proximal movement of the stopper 25 expels the drug 24 from the reservoir of the drug storage container 23 into the delivery member 16. The distal end of the drug storage container 23 may be open to allow a plunger 26 to extend into the drug storage container 23 and push the stopper 25 in the proximal direction. In the present embodiment, the plunger 26 and the stopper 25 are initially spaced from each other by a gap. Upon activation of a drive assembly 30, the plunger 26 moves in the proximal direction to close the gap and comes into contact with the stopper 25. Subsequent proximal movement of the plunger 26 drives the stopper 25 in the proximal direction. In alternative embodiments, the stopper 25 and the plunger 26 may be coupled to each other, e.g., via a threaded coupling, such that they move together jointly from the start of movement of the plunger 26. Once the stopper 25 is in motion, it may continue to move in the proximal direction until it contacts a distally-facing portion of the interior surface of the wall of the drug storage container 23. This position of the stopper 25 may be referred to as the end-of-dose position and may correspond to when delivery of the drug 24 to the patient is complete or substantially complete.

The delivery member 16 is connected or operable to be connected in fluid communication with the reservoir of the drug storage container 23. A proximal end of the delivery member 16 may define the insertion end 16a of the delivery member 16. The insertion end 16a may include a sharpened tip of other pointed geometry allowing the insertion end 16a to pierce the patient's skin and subcutaneous tissue during insertion of the delivery member 16. The delivery member 16 may be hollow and have an interior passageway. One or more openings may be formed in the insertion end 16a to allow drug to flow out of the delivery member 16 into the patient.

In the present embodiment, the drug storage container 23 is a pre-filled syringe and has a staked, hollow metal needle for the delivery member 16. Here, the needle is fixed relative to the wall of the drug storage container 23 and is in permanent fluid communication with the reservoir of the drug storage container 23. In other embodiments, the drug storage container 23 may be a needle-less cartridge, and, as such, initially may not be in fluid communication with the delivery member 16. In such embodiments, the drug storage container 23 may move toward a distal end of the delivery member 16, or vice versa, during operation of the drug delivery device 10 such that the distal end of the delivery member 16 penetrates through a septum covering an opening in the drug storage container 23 thereby establishing fluid communication with the reservoir of the drug storage container 23.

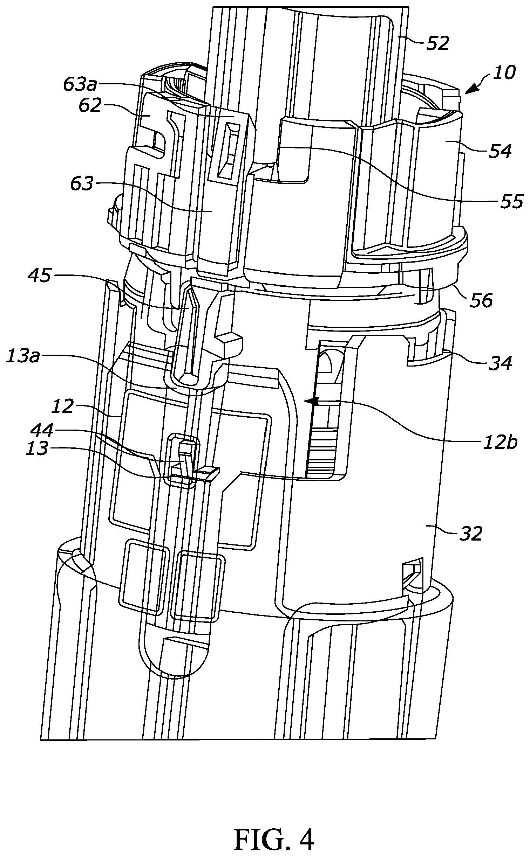

The drug storage container 23 may be fixed relative to the housing 12 such that the drug storage container 23 does not move relative to the housing 12 once installed therein. As such, the insertion end 16a of the delivery member 16 may extend permanently through the opening 14 in the housing 12 in the pre-delivery, delivery, and post-delivery states. In the present embodiment, a container holder 42 fixes the position of the drug storage container 23 within the housing 12. The container holder 42 may have a hollow and generally cylindrical or tubular shape, and the drug storage container 23 may be disposed partially or entirely within the container holder 42. A proximal end of the container holder 42 may include an inwardly protruding flange 42a abutting against a neck of the drug storage container 23, thereby preventing proximal movement of the drug storage container 23. In some, but not all approaches, the container holder 42 may be fixedly attached to the housing 12 such that the container holder 42 is prevented from moving relative to the housing 12 during operation of the drug delivery device 10. In these and other examples, the container holder 42 may be operably attached to the housing 12 via other components such as, for example, the nut (which will be discussed in further detail below). A distal end of the container holder 42 may include a housing coupling 44 and at least one arm 45. More specifically, the housing coupling 44 is in the form of a number of tabs or protrusions that are dimensioned to engage and be operably coupled with a portion of the housing 12. As an example, and as illustrated in Fig. 4, the distal end 12b of the housing 12 may include an opening 13 and a slot 13a that engages the housing coupling 44. So arranged, the container holder 42 is fixedly attached with the housing 12 such that they may both move in unison in the axial direction.

In alternative embodiments, the drug storage container 23 may be moveably coupled to the housing 12 such that the drug storage container 23 is able to move relative to the housing 12 during operation of the drug delivery device 10. In certain such alternative embodiments, the insertion end 16a of the delivery member 16 may be retracted within the opening 14 in the housing 12 in the pre-delivery state. Subsequently, during operation of the injection device 10, the insertion end 16a of the delivery member 16 may be deployed through the opening 14 in the housing 12 for insertion into the patient. This motion may, in some embodiments, be the result of the drug storage container 23 having been driven in the proximal direction relative to the housing 12.

The plunger 26 may be constructed in multiple, interconnected pieces, or alternatively, have a one-piece construction. In the present embodiment, the plunger 26 includes a rod 65 having a threaded outer surface 66 and washer or disk 68 rigidly attached to a proximal end of the rod 65. The disk 68 may impact and push the stopper 25 when the drive assembly 30 is activated. Accordingly, in some embodiments, the disk 68 may have shock-absorbing properties to attenuate any shock or vibrations associated with the impact event.

The drug delivery device 10 may further include a guard mechanism for preventing contact with the insertion end 16a of the delivery member 16 when the drug delivery device 10 is not being used to administer an injection. The guard mechanism may include a shield 32 moveably disposed at the proximal end 12a of the housing 12 adjacent to the opening 14. The shield 32 may have a hollow and generally cylindrical or tubular shape. The shield 32 may have a distal end received within the housing 12, and may be configured to move relative to the housing 12 between an extended position wherein a proximal end of the shield 32 extends through the opening 14 in the housing 12 and a retracted position wherein the proximal end of the shield 32 is retracted, fully or partially, into the opening 14 in the housing 12. In at least the extended position, the shield 32 may extend beyond and surround the insertion end 16a of the delivery member 16. In some embodiments, moving the shield 32 toward the retracted position may expose the insertion end 16a of the delivery member 16. Further, in some embodiments, the shield 32 may be coupled to the housing 12 and/or the container holder 42 via, for example, a pin-and-slot arrangement such that the shield 32 is able to translate in a linear direction relative to the housing 12 and/or the container holder 42 but is prevented from rotating relative to the housing 12 and/or the container holder 42.

The proximal end of the shield 32 may include a skin contacting portion 36 (Fig. 2). With reference to Fig. 4, the distal end of the shield 32 may include an activator portion 34. In some examples, the detent activator portion 34 and the skin-contacting portion 36 may be integrally formed to define a single, monolithic structure. At least the skin-contacting portion 36 of shield 32 may have a hollow and generally cylindrical or tubular shape and, in some embodiments, may be centered about the longitudinal axis A of the drug delivery device 10. The activator portion 34 of the shield may be a cutout or recessed region, and will be discussed in further detail below.

Moving the shield 32 from the extended position to the retracted position may be accomplished by pressing the skin-contacting portion 36 against the patient's skin at the injection site. In examples where the delivery member 16 protrudes from the opening 14 in the housing 12 in the pre-delivery or storage state, this motion may result in the insertion end 16a of the delivery member 16 being inserted into the patient's skin.

The guard mechanism may further include a guard biasing member 35. The guard biasing member 35 may bias or urge the guard 32 towards the extended position by exerting a biasing force in the proximal direction on the shield 32. In some examples, the guard biasing member 35 is in the form of a compression spring. In other examples (not illustrated), the guard biasing member 35 may be in the form of a torsion or other form of spring. In any event, a user may overcome this biasing force by pressing the shield 32 against the injection site. When the injection is complete and the drug delivery device 10 is lifted off of the injection site, the guard biasing member 35 may return the shield 32 to the extended position, thereby covering the insertion end 16a of the deliver member 16. In some embodiments, the guard biasing member 35 may be positioned in the axial direction between, and in contact with both, a distally facing inner surface of the shield 32 and a proximally facing inner or outer surface of a lock 40. In embodiments where the shield 32 is a compression spring, movement of the shield 32 in the distal direction may cause the guard biasing member 35 to be compressed between the shield 32 and the lock 40. In some embodiments, the guard biasing member 35 may be partially compressed prior to retraction of the shield 32 and thus exert a biasing force on both the shield 32 and the lock 40 in the pre-delivery state.

As previously noted, the drug delivery device 10 may further include the drive assembly 30 disposed partially or entirely within the housing 12. Generally, the drive assembly 30 may be configured to store energy and, upon or in response to activation of the drive assembly 30 by the user, release or output that energy to drive the injection assembly 15 (i.e., the delivery member 16, the drug storage container 23, the stopper 25, and the plunger 26) to expel the drug 24 from the drug storage container 23 through the delivery member 16 into the patient. In the present example, the drive assembly 30 is configured to store mechanical potential energy; however, alternative embodiments of the drive assembly 30 may be configured differently, with, for example, the drive assembly 30 storing electrical or chemical potential energy. Upon activation of the drive assembly 30, the drive assembly 30 may convert the potential energy into kinetic energy for moving the plunger 26.

Generally, the drive assembly 30 may include a rotational biasing member 50, a rotational biasing member housing 52, a trigger ring 54, and a mechanical linkage 58. The rotational biasing member 50 may be a torsion spring (e.g., a spiral torsion, a helical torsion spring, etc.) which is initially retained in an energized state. In the energized state, the rotational biasing member 50 may be twisted or wound and retained in that twisted or wound configuration by the trigger ring 54 via the mechanical linkage 58. When released, the rotational biasing member 50 will try to return to its natural length or shape, and as a result, exert a biasing force causing the mechanical linkage 58 to rotate. The mechanical linkage 58, in turn, may convert the rotational motion into linear motion for driving the plunger 26 in the proximal direction. In some embodiments, the mechanical linkage 58 may convert the rotational motion from the rotational biasing member 50 into linear motion for driving the plunger 26 in the proximal direction and rotational motion of the plunger 26 about the longitudinal axis A.

Alternative embodiments may utilize an energy source different from a rotational biasing member. Certain alternative embodiments may utilize, for example, a linear biasing member (e.g., a helical compression spring, a helical extension spring, etc.) which, when released, outputs a force in the direction of travel of the plunger 26. In addition to or as an alternative to a biasing member, other embodiments may include any one or combination of: an electromechanical arrangement including an electric motor and/or solenoid and a drive train or transmission coupled to the plunger 26; or an arrangement that generates or releases a pressurized gas or fluid to propel the plunger 26 or which acts directly on the stopper 25 to move stopper 25 through the drug storage container 23 to expel the drug 24 from therein. In embodiments where the drug storage container 23 and/or the delivery member 16 is moveable relative to the housing 12, the drive assembly 30 may, upon activation, drive the drug storage container 23 and/or the delivery member 16 in the proximal direction so as to cause the insertion end 16a of the delivery member 16 to be inserted into the patient. Thus, in certain embodiments, the drive assembly 30 may provide the motive force needed for both inserting the delivery member 16 into the patient and expelling the drug 24 from the drug storage container 23.

As illustrated in Fig. 4, the trigger ring 54 may include an arm opening 55. The arm opening 55 may be in the form of a cutout or recess formed in the body of the arm opening 55. The trigger ring 54 may additionally include an activator portion 56. In the illustrated example, the activator portion 56 is in the form of a tab positioned on a proximal end of the trigger ring 54.

The mechanical linkage 58 may include a plunger guide 60 and a nut 62. The plunger guide 60 may have a hollow and generally cylindrical or tubular shape. The distal end of the plunger 26 may be disposed inside of the plunger guide 60 in at least the pre-delivery state. A distal extend of the plunger guide 60 may extend through the center of the rotational biasing member 50 and may be coupled to the rotational biasing member 50 such that the plunger guide 60 rotates jointly together with the rotational biasing member 50 when the rotational biasing member 50 is released. An inner surface of the plunger guide 60 is coupled to an outer surface of the plunger 26 such that the plunger 26 rotates jointly together with plunger guide 60 when the rotational biasing member 50 is released, while permitting axial movement of the plunger 26 relative to the plunger guide 60. The coupling between the plunger guide 60 and the plunger 26 may be achieved via, for example, a splined arrangement, wherein a longitudinal protrusion on one of the inner surface of the plunger guide 60 or the outer surface of the plunger 26 is slidably received in a longitudinal slot on the other one of the outer surface of the plunger 26 or the inner surface of the plunger guide 60.

The nut 62 may have a generally annular shape and may be disposed around a proximal end of the plunger 26 in the pre-delivery state. Further, as illustrated in Fig. 4, a portion of the nut 62 may at least partially surround a portion of the trigger ring 54. The nut 62 may be fixedly mounted such that the nut 62 is immoveable relative to the housing 12. Furthermore, the nut 62 may have a threaded inner surface 64 which engages the threaded outer surface 66 of the plunger 26. As a consequence of this threaded engagement, rotation of the plunger 26 relative to the nut 62 may drive the plunger 26 linearly in the proximal direction. This in turn causes the plunger 26 to act on and push the stopper in the proximal direction to expel the drug 24 from the storage container 23 into the patient via the inserted delivery member 16. The nut 62 may further include at least one arm 63 that extends in a distal direction from the nut 62. In some examples, the at least one arm 63 may be in the form of a peak force arm that may generate a force peak during intended activation. The at least one arm 63 may include a finger 63a at its distal end that, in a pre-activated state, is positioned adjacent to the arm opening 55 of the trigger ring 54. In some examples, the at least one arm 63 may be constructed from a resilient and/or flexible material that is biased to a position that does not engage or contact a portion of the trigger ring 54. Further, in some examples and as illustrated in Fig. 4, the arm or arms 45 of the container holder 42 are positioned adjacent to the at least one arm or arms 63 in the pre-activated state.

The shield 32 may be configured to interact with the drive assembly 30 when the shield 32 moves from the extended position to the retracted position. This interaction may activate the drive assembly 30 to output the energy needed for driving the plunger 26 to expel the drug 24 from the drug storage container 23 and/or insert the insertion end 16a of the delivery member 16 into the patient's skin. In the present embodiment, movement of the shield 32 from the extended position to the retracted position releases the rotational biasing member 50 from the energized state, thereby allowing the rotational biasing member 50 to deenergize and drive the plunger 26, via the mechanical linkage 58, to expel the drug 24 from the drug storage container 23. More particularly, in the pre-delivery state, the trigger ring 54 may be arranged in an initial position where it lockingly engages an exterior surface of the plunger guide 60, thereby preventing the plunger guide 60 from rotating under the biasing force of the rotational biasing member 50. As a consequence, the rotational biasing member 50 is prevented de-energizing. When the shield 32 moves from the extended position to the retracted position as a result of being pressed against the patient's skin, the activator portion 34 of the shield 32 engages the activator portion 56 of the trigger ring to urge the trigger ring 54 in the distal direction to a releasing position where the trigger ring 54 disengages from the plunger guide 60. More specifically, in these and other examples, the recessed activator portion 34 of the trigger ring may at least partially surround the tabbed activator portion 56 to form a closely-fit coupling therebetween. As a consequence, the plunger guide 60 is able to rotate under the biasing force of the rotational biasing member 50 and drive, via the threaded connection between the plunger 26 and the nut 62, the plunger 26 in the proximal direction.

The rotational biasing member housing 52 may be disposed within and rigidly attached to the housing 12. The rotational biasing member housing 52 may have a hollow and generally cylindrical or tubular shape, and may receive, in full or in part, the rotational biasing member 52 such that the rotational biasing member housing 52 surrounds or partially surrounds the rotational biasing member 50. The rotational biasing member housing 52 may serve as a mount or seat for the rotational biasing member 50 to push off of when released.

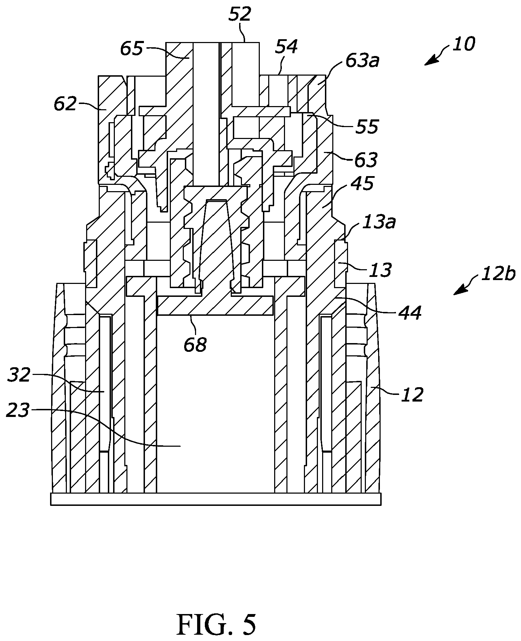

Having described the general configuration and operation of the drug delivery device 10, it is appreciated that axial movement of the shield 32 towards the distal end 12b of the housing 12 serves to activate the drive assembly 30 to deliver the drug 24 via the injection assembly 15. However, it may be the case that at some point during the pre-activated state, a user may inadvertently drop or jostle the drug delivery device 10 in a way that the housing and/or the shield are urged towards the distal end 12b of the housing 12. As illustrated in Figs. 4 and 5, in the pre-activated state, the finger 63a of the at least one arm 63 of the nut 62 is positioned adjacent to the arm opening 55 of the trigger ring 54, while the arm 45 of the container holder 42 is positioned adjacent to the at least one arm 63 of the nut 62. To prepare for drug administration, a user may pull the cap 18 in the proximal direction 12a away from the device 10 to expose the skin-contacting portion 36 of the shield 32 to engage a patient's skin.

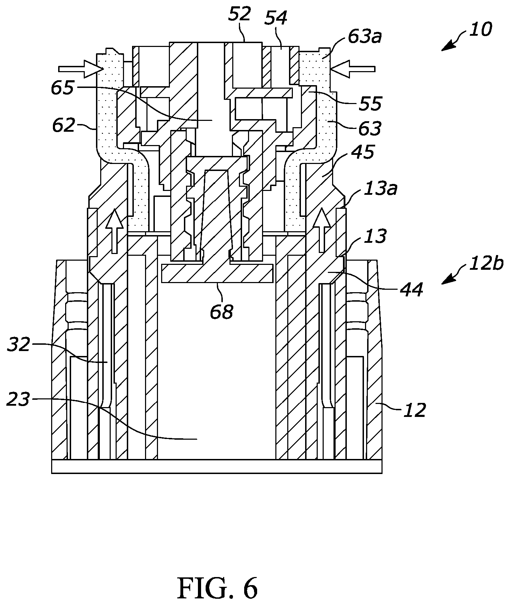

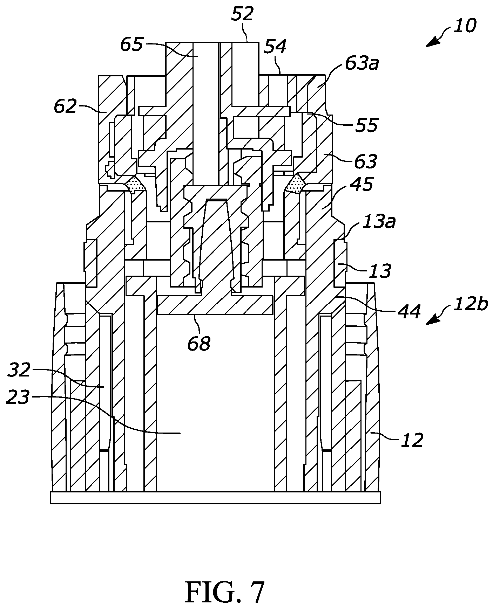

Prior to a user deciding to proceed with drug administration (i.e., either before or after removing the cap 18), the container holder 42, the nut 62, and the trigger ring 54 cooperate to form a retention mechanism that prevents the drive assembly 30 from activating. More specifically, with reference to Figs. 5-7 in the event of an accidental drop and/or jostling of the device 10, inertial and/or contact forces may cause the housing (and thus the container holder 42 coupled therewith) to move in the axial direction towards the distal end 12b of the housing 12. As illustrated in Fig. 6, upon such movement occurring, the arm 45 of the container holder slidingly engages or otherwise urges the at least one arm 63 of the nut 62 inwards in a radial direction towards the longitudinal axis A, which in turn urges the at least one arm inwards in the radial direction towards the longitudinal axis A. This relative movement causes the finger 63a of the at least one arm 63 to engage the arm opening 55 formed on the trigger ring 54 and be at least partially inserted therein, and as such, the nut 62 and container holder 42 restrict or prevent the trigger ring 54from further advancing axially towards the distal end 12b of the housing.

With reference to Fig. 8, after a period of time, the internal components will stop moving relative to each other, returning to their default location, and the arm 45 of the container holder, along with the at least one arm 63, will disengage from the opening 55 formed on the trigger ring 54, thereby permitting the device 10 to be used as desired (i.e., remaining in the pre-activated state and/or removing the cap 18 to transition to the delivery or dosing state. Here, the user may pull and detach the removable cap 18 from the housing 12. This may uncover the insertion end 16a of the delivery member 16. Nevertheless, the insertion end 16a of the delivery member 16 will remain surrounded by the shield 32 at this stage. The user may position the skin-contacting portion 36 of the shield 32 over the desired injection site and then push the skin-contacting portion 36 against the injection site. The force applied by the user will overcome the biasing force of the guard biasing member 35, thereby causing the shield 32 to retract into the opening 14 moving from the extended position to the retracted position in the distal direction. Notably, when the device 10 is used as intended, the housing 12 and the container holder 42 do not engage the nut, and as such, movement of the shield 32 to urge the trigger ring 54 is permitted. The delivery member 16 remains stationary relative to the housing 12 during the retracting movement of the shield 32.

The retraction of the shield 32 may cause any number of actions to occur. Because the delivery member 16 remains stationary relative to the housing 12 during retraction of the shield 32, the insertion end 16a of the delivery member 16 is caused to protrude through an opening in the skin-contacting portion 36 of the shield 32 and thereby pierce the patient's skin at the injection site and penetrate into the patient's subcutaneous tissue. As previously noted, retraction of the shield 32 activates the drive assembly 30. More particularly, retraction of the shield 32 may cause the activator portion 34 to engage the arm opening 55 of the trigger ring 54 to move the trigger ring 54 in the distal direction to the releasing position where the trigger ring 54 disengages from the plunger guide 60, thereby activating the drive assembly 30 to deliver the drug 24 via the injection assembly 15.

So configured, the retention mechanism serves to prevent inadvertent activation of the device if it is dropped. The arm 45 of the container holder urges the at least one arm 63 of the nut 62 inwards and clamps onto the trigger ring 54 in the event the container holder 42 travels excessively far into the device 10 in the distal direction. Such a retention mechanism removes energy from the device to allow the device to return to its default, pre-activated state.

The above description describes various devices, assemblies, components, subsystems and methods for use related to a drug delivery device. The devices, assemblies, components, subsystems, methods or drug delivery devices can further comprise or be used with a drug including but not limited to those drugs identified below as well as their generic and biosimilar counterparts. The term drug, as used herein, can be used interchangeably with other similar terms and can be used to refer to any type of medicament or therapeutic material including traditional and non-traditional pharmaceuticals, nutraceuticals, supplements, biologics, biologically active agents and compositions, large molecules, biosimilars, bioequivalents, therapeutic antibodies, polypeptides, proteins, small molecules and generics. Non-therapeutic injectable materials are also encompassed. The drug may be in liquid form, a lyophilized form, or in a reconstituted from lyophilized form. The following example list of drugs should not be considered as all-inclusive or limiting.

The drug will be contained in a reservoir. In some instances, the reservoir is a primary container that is either filled or pre-filled for treatment with the drug. The primary container can be a vial, a cartridge or a pre-filled syringe.

In some embodiments, the reservoir of the drug delivery device may be filled with or the device can be used with colony stimulating factors, such as granulocyte colony-stimulating factor (G-CSF). Such G-CSF agents include but are not limited to Neulasta^{®} (pegfilgrastim, pegylated filgastrim, pegylated G-CSF, pegylated hu-Met-G-CSF) and Neupogen^{®} (filgrastim, G-CSF, hu-MetG-CSF), UDENYCA^{®} (pegfilgrastim-cbqv), Ziextenzo^{®} (LA-EP2006; pegfilgrastim-bmez), or FULPHILA (pegfilgrastim-bmez).

In other embodiments, the drug delivery device may contain or be used with an erythropoiesis stimulating agent (ESA), which may be in liquid or lyophilized form. An ESA is any molecule that stimulates erythropoiesis. In some embodiments, an ESA is an erythropoiesis stimulating protein. As used herein, "erythropoiesis stimulating protein" means any protein that directly or indirectly causes activation of the erythropoietin receptor, for example, by binding to and causing dimerization of the receptor. Erythropoiesis stimulating proteins include erythropoietin and variants, analogs, or derivatives thereof that bind to and activate erythropoietin receptor; antibodies that bind to erythropoietin receptor and activate the receptor; or peptides that bind to and activate erythropoietin receptor. Erythropoiesis stimulating proteins include, but are not limited to, Epogen^{®} (epoetin alfa), Aranesp^{®} (darbepoetin alfa), Dynepo^{®} (epoetin delta), Mircera^{®} (methyoxy polyethylene glycol-epoetin beta), Hematide^{®}, MRK-2578, INS-22, Retacrit^{®} (epoetin zeta), Neorecormon^{®} (epoetin beta), Silapo^{®} (epoetin zeta), Binocrit^{®} (epoetin alfa), epoetin alfa Hexal, Abseamed^{®} (epoetin alfa), Ratioepo^{®} (epoetin theta), Eporatio^{®} (epoetin theta), Biopoin^{®} (epoetin theta), epoetin alfa, epoetin beta, epoetin iota, epoetin omega, epoetin delta, epoetin zeta, epoetin theta, and epoetin delta, pegylated erythropoietin, carbamylated erythropoietin, as well as the molecules or variants or analogs thereof.

Among particular illustrative proteins are the specific proteins set forth below, including fusions, fragments, analogs, variants or derivatives thereof: OPGL specific antibodies, peptibodies, related proteins, and the like (also referred to as RANKL specific antibodies, peptibodies and the like), including fully humanized and human OPGL specific antibodies, particularly fully humanized monoclonal antibodies; Myostatin binding proteins, peptibodies, related proteins, and the like, including myostatin specific peptibodies; IL-4 receptor specific antibodies, peptibodies, related proteins, and the like, particularly those that inhibit activities mediated by binding of IL-4 and/or IL-13 to the receptor; Interleukin 1-receptor 1 ("IL1-R1") specific antibodies, peptibodies, related proteins, and the like; Ang2 specific antibodies, peptibodies, related proteins, and the like; NGF specific antibodies, peptibodies, related proteins, and the like; CD22 specific antibodies, peptibodies, related proteins, and the like, particularly human CD22 specific antibodies, such as but not limited to humanized and fully human antibodies, including but not limited to humanized and fully human monoclonal antibodies, particularly including but not limited to human CD22 specific IgG antibodies, such as, a dimer of a human-mouse monoclonal hLL2 gamma-chain disulfide linked to a human-mouse monoclonal hLL2 kappa-chain, for example, the human CD22 specific fully humanized antibody in Epratuzumab, CAS registry number 501423-23-0; IGF-1 receptor specific antibodies, peptibodies, and related proteins, and the like including but not limited to anti-IGF-1R antibodies; B-7 related protein 1 specific antibodies, peptibodies, related proteins and the like ("B7RP-1" and also referring to B7H2, ICOSL, B7h, and CD275), including but not limited to B7RP-specific fully human monoclonal IgG2 antibodies, including but not limited to fully human IgG2 monoclonal antibody that binds an epitope in the first immunoglobulin-like domain of B7RP-1, including but not limited to those that inhibit the interaction of B7RP-1 with its natural receptor, ICOS, on activated T cells; IL-15 specific antibodies, peptibodies, related proteins, and the like, such as, in particular, humanized monoclonal antibodies, including but not limited to HuMax IL-15 antibodies and related proteins, such as, for instance, 145c7; IFN gamma specific antibodies, peptibodies, related proteins and the like, including but not limited to human IFN gamma specific antibodies, and including but not limited to fully human anti-IFN gamma antibodies; TALL-1 specific antibodies, peptibodies, related proteins, and the like, and other TALL specific binding proteins; Parathyroid hormone ("PTH") specific antibodies, peptibodies, related proteins, and the like; Thrombopoietin receptor ("TPO-R") specific antibodies, peptibodies, related proteins, and the like;Hepatocyte growth factor ("HGF") specific antibodies, peptibodies, related proteins, and the like, including those that target the HGF/SF:cMet axis (HGF/SF:c-Met), such as fully human monoclonal antibodies that neutralize hepatocyte growth factor/scatter (HGF/SF); TRAIL-R2 specific antibodies, peptibodies, related proteins and the like; Activin A specific antibodies, peptibodies, proteins, and the like; TGF-beta specific antibodies, peptibodies, related proteins, and the like; Amyloid-beta protein specific antibodies, peptibodies, related proteins, and the like; c-Kit specific antibodies, peptibodies, related proteins, and the like, including but not limited to proteins that bind c-Kit and/or other stem cell factor receptors; OX40L specific antibodies, peptibodies, related proteins, and the like, including but not limited to proteins that bind OX40L and/or other ligands of the OX40 receptor; Activase^{®} (alteplase, tPA); Aranesp^{®} (darbepoetin alfa) Erythropoietin [30-asparagine, 32-threonine, 87-valine, 88-asparagine, 90-threonine], Darbepoetin alfa, novel erythropoiesis stimulating protein (NESP); Epogen^{®} (epoetin alfa, or erythropoietin); GLP-1, Avonex^{®} (interferon beta-1a); Bexxar^{®} (tositumomab, anti-CD22 monoclonal antibody); Betaseron^{®} (interferon-beta); Campath^{®} (alemtuzumab, anti-CD52 monoclonal antibody); Dynepo^{®} (epoetin delta); Velcade^{®} (bortezomib); MLN0002 (anti-α4ß7 mAb); MLN1202 (anti-CCR2 chemokine receptor mAb); Enbrel^{®} (etanercept, TNF-receptor /Fc fusion protein, TNF blocker); Eprex^{®} (epoetin alfa); Erbitux^{®} (cetuximab, anti-EGFR / HER1 / c-ErbB-1); Genotropin^{®} (somatropin, Human Growth Hormone); Herceptin^{®} (trastuzumab, anti-HER2/neu (erbB2) receptor mAb); Kanjinti^{™} (trastuzumab-anns) anti-HER2 monoclonal antibody, biosimilar to Herceptin^{®}, or another product containing trastuzumab for the treatment of breast or gastric cancers; Humatrope^{®} (somatropin, Human Growth Hormone); Humira^{®} (adalimumab); Vectibix^{®} (panitumumab), Xgeva^{®} (denosumab), Prolia^{®} (denosumab), Immunoglobulin G2 Human Monoclonal Antibody to RANK Ligand, Enbrel^{®} (etanercept, TNF-receptor /Fc fusion protein, TNF blocker), Nplate^{®} (romiplostim), rilotumumab, ganitumab, conatumumab, brodalumab, insulin in solution; Infergen^{®} (interferon alfacon-1); Natrecor^{®} (nesiritide; recombinant human B-type natriuretic peptide (hBNP); Kineret^{®} (anakinra); Leukine^{®} (sargamostim, rhuGM-CSF); LymphoCide^{®} (epratuzumab, anti-CD22 mAb); Benlysta^{™} (lymphostat B, belimumab, anti-BlyS mAb); Metalyse^{®} (tenecteplase, t-PA analog); Mircera^{®} (methoxy polyethylene glycol-epoetin beta); Mylotarg^{®} (gemtuzumab ozogamicin); Raptiva^{®} (efalizumab); Cimzia^{®} (certolizumab pegol, CDP 870); Soliris^{™} (eculizumab); pexelizumab (anti-C5 complement); Numax^{®} (MEDI-524); Lucentis^{®} (ranibizumab); Panorex^{®} (17-1A, edrecolomab); Trabio^{®} (lerdelimumab); TheraCim hR3 (nimotuzumab); Omnitarg (pertuzumab, 2C4); Osidem^{®} (IDM-1); OvaRex^{®} (B43.13); Nuvion^{®} (visilizumab); cantuzumab mertansine (huC242-DM1); NeoRecormon^{®} (epoetin beta); Neumega^{®} (oprelvekin, human interleukin-11); Orthoclone OKT3^{®} (muromonab-CD3, anti-CD3 monoclonal antibody); Procrit^{®} (epoetin alfa); Remicade^{®} (infliximab, anti-TNFα monoclonal antibody); Reopro^{®} (abciximab, anti-GP IIb/IIia receptor monoclonal antibody); Actemra^{®} (anti-IL6 Receptor mAb); Avastin^{®} (bevacizumab), HuMax-CD4 (zanolimumab); MvasiTM (bevacizumabawwb); Rituxan^{®} (rituximab, anti-CD20 mAb); Tarceva^{®} (erlotinib); Roferon-A^{®}-(interferon alfa-2a); Simulect^{®} (basiliximab); Prexige^{®} (lumiracoxib); Synagis^{®} (palivizumab); 145c7-CHO (anti-IL15 antibody, see U.S. Patent No. 7,153,507); Tysabri^{®} (natalizumab, anti-α4integrin mAb); Valortim^{®} (MDX-1303, anti-B. anthracis protective antigen mAb); ABthrax^{™}; Xolair^{®} (omalizumab); ETI211 (anti-MRSA mAb); IL-1 trap (the Fc portion of human IgG1 and the extracellular domains of both IL-1 receptor components (the Type I receptor and receptor accessory protein)); VEGF trap (Ig domains of VEGFR1 fused to IgG1 Fc); Zenapax^{®} (daclizumab); Zenapax^{®} (daclizumab, anti-IL-2Rα mAb); Zevalin^{®} (ibritumomab tiuxetan); Zetia^{®} (ezetimibe); Orencia^{®} (atacicept, TACI-Ig); anti-CD80 monoclonal antibody (galiximab); anti-CD23 mAb (lumiliximab); BR2-Fc (huBR3 / huFc fusion protein, soluble BAFF antagonist); CNTO 148 (golimumab, anti-TNFα mAb); HGS-ETR1 (mapatumumab; human anti-TRAIL Receptor-1 mAb); HuMax-CD20 (ocrelizumab, anti-CD20 human mAb); HuMax-EGFR (zalutumumab); M200 (volociximab, anti-a5β1 integrin mAb); MDX-010 (ipilimumab, anti-CTLA-4 mAb and VEGFR-1 (IMC-18F1); anti-BR3 mAb; anti-C. difficile Toxin A and Toxin B C mAbs MDX-066 (CDA-1) and MDX-1388); anti-CD22 dsFv-PE38 conjugates (CAT-3888 and CAT-8015); anti-CD25 mAb (HuMax-TAC); anti-CD3 mAb (NI-0401); adecatumumab; anti-CD30 mAb (MDX-060); MDX-1333 (anti-IFNAR); anti-CD38 mAb (HuMax CD38); anti-CD40L mAb; anti-Cripto mAb; anti-CTGF Idiopathic Pulmonary Fibrosis Phase I Fibrogen (FG-3019); anti-CTLA4 mAb; anti-eotaxin1 mAb (CAT-213); anti-FGF8 mAb; anti-ganglioside GD2 mAb; anti-ganglioside GM2 mAb; anti-GDF-8 human mAb (MYO-029); anti-GM-CSF Receptor mAb (CAM-3001); anti-HepC mAb (HuMax HepC); anti-IFNα mAb (MEDI-545, MDX-198); anti-IGF1 R mAb; anti-IGF-1R mAb (HuMax-Inflam); anti-IL12 mAb (ABT-874); anti-IL12/IL23 mAb (CNTO 1275); anti-IL13 mAb (CAT-354); anti-IL2Ra mAb (HuMax-TAC); anti-IL5 Receptor mAb; anti-integrin receptors mAb (MDX-018, CNTO 95); anti-IP10 Ulcerative Colitis mAb (MDX-1100); BMS-66513; anti-Mannose Receptor/hCGβ mAb (MDX-1307); anti-mesothelin dsFv-PE38 conjugate (CAT-5001); anti-PD1mAb (MDX-1106 (ONO-4538)); anti-PDGFRα antibody (IMC-3G3); anti-TGFß mAb (GC-1008); anti-TRAIL Receptor-2 human mAb (HGS-ETR2); anti-TWEAK mAb; anti-VEGFR/Flt-1 mAb; and anti-ZP3 mAb (HuMax-ZP3).

In some embodiments, the drug delivery device may contain or be used with a sclerostin antibody, such as but not limited to romosozumab, blosozumab, BPS 804 (Novartis), Evenity^{™} (romosozumab-aqqg), another product containing romosozumab for treatment of postmenopausal osteoporosis and/or fracture healing and in other embodiments, a monoclonal antibody (IgG) that binds human Proprotein Convertase Subtilisin/Kexin Type 9 (PCSK9). Such PCSK9 specific antibodies include, but are not limited to, Repatha^{®} (evolocumab) and Praluent^{®} (alirocumab). In other embodiments, the drug delivery device may contain or be used with rilotumumab, bixalomer, trebananib, ganitumab, conatumumab, motesanib diphosphate, brodalumab, vidupiprant or panitumumab. In some embodiments, the reservoir of the drug delivery device may be filled with or the device can be used with IMLYGIC^{®} (talimogene laherparepvec) or another oncolytic HSV for the treatment of melanoma or other cancers including but are not limited to OncoVEXGALV/CD; OrienX010; G207, 1716; NV1020; NV12023; NV1034; and NV1042. In some embodiments, the drug delivery device may contain or be used with endogenous tissue inhibitors of metalloproteinases (TIMPs) such as but not limited to TIMP-3. In some embodiments, the drug delivery device may contain or be used with Aimovig^{®} (erenumab-aooe), anti-human CGRP-R (calcitonin gene-related peptide type 1 receptor) or another product containing erenumab for the treatment of migraine headaches. Antagonistic antibodies for human calcitonin gene-related peptide (CGRP) receptor such as but not limited to erenumab and bispecific antibody molecules that target the CGRP receptor and other headache targets may also be delivered with a drug delivery device of the present disclosure. Additionally, bispecific T cell engager (BiTE^{®}) molecules such as but not limited to BLINCYTO^{®} (blinatumomab) can be used in or with the drug delivery device of the present disclosure. In some embodiments, the drug delivery device may contain or be used with an APJ large molecule agonist such as but not limited to apelin or analogues thereof. In some embodiments, a therapeutically effective amount of an anti-thymic stromal lymphopoietin (TSLP) or TSLP receptor antibody is used in or with the drug delivery device of the present disclosure. In some embodiments, the drug delivery device may contain or be used with AvsolaTM (infliximab-axxq), anti-TNF α monoclonal antibody, biosimilar to Remicade^{®} (infliximab) (Janssen Biotech, Inc.) or another product containing infliximab for the treatment of autoimmune diseases. In some embodiments, the drug delivery device may contain or be used with Kyprolis^{®} (carfilzomib), (2S)-N-((S)-1-((S)-4-methyl-1-((R)-2-methyloxiran-2-yl)-1-oxopentan-2-ylcarbamoyl)-2-phenylethyl)-2-((S)-2-(2-morpholinoacetamido)-4-phenylbutanamido)-4-methylpentanamide, or another product containing carfilzomib for the treatment of multiple myeloma. In some embodiments, the drug delivery device may contain or be used with Otezla^{®} (apremilast), N-[2-[(1S)-1-(3-ethoxy-4-methoxyphenyl)-2-(methylsulfonyl)ethyl]-2,3-dihydro-1,3-dioxo- 1H-isoindol-4-yl]acetamide, or another product containing apremilast for the treatment of various inflammatory diseases. In some embodiments, the drug delivery device may contain or be used with ParsabivTM (etelcalcetide HCl, KAI-4169) or another product containing etelcalcetide HCl for the treatment of secondary hyperparathyroidism (sHPT) such as in patients with chronic kidney disease (KD) on hemodialysis. In some embodiments, the drug delivery device may contain or be used with ABP 798 (rituximab), a biosimilar candidate to Rituxan^{®}/MabThera^{™}, or another product containing an anti-CD20 monoclonal antibody. In some embodiments, the drug delivery device may contain or be used with a VEGF antagonist such as a non-antibody VEGF antagonist and/or a VEGF-Trap such as aflibercept (Ig domain 2 from VEGFR1 and Ig domain 3 from VEGFR2, fused to Fc domain of IgG1). In some embodiments, the drug delivery device may contain or be used with ABP 959 (eculizumab), a biosimilar candidate to Soliris^{®}, or another product containing a monoclonal antibody that specifically binds to the complement protein C5. In some embodiments, the drug delivery device may contain or be used with Rozibafusp alfa (formerly AMG 570) is a novel bispecific antibody-peptide conjugate that simultaneously blocks ICOSL and BAFF activity. In some embodiments, the drug delivery device may contain or be used with Omecamtiv mecarbil, a small molecule selective cardiac myosin activator, or myotrope, which directly targets the contractile mechanisms of the heart, or another product containing a small molecule selective cardiac myosin activator. In some embodiments, the drug delivery device may contain or be used with Sotorasib (formerly known as AMG 510), a KRASG12C small molecule inhibitor, or another product containing a KRASG12C small molecule inhibitor. In some embodiments, the drug delivery device may contain or be used with Tezepelumab, a human monoclonal antibody that inhibits the action of thymic stromal lymphopoietin (TSLP), or another product containing a human monoclonal antibody that inhibits the action of TSLP. In some embodiments, the drug delivery device may contain or be used with AMG 714, a human monoclonal antibody that binds to Interleukin-15 (IL-15) or another product containing a human monoclonal antibody that binds to Interleukin-15 (IL-15). In some embodiments, the drug delivery device may contain or be used with AMG 890, a small interfering RNA (siRNA) that lowers lipoprotein(a), also known as Lp(a), or another product containing a small interfering RNA (siRNA) that lowers lipoprotein(a). In some embodiments, the drug delivery device may contain or be used with ABP 654 (human IgG1 kappa antibody), a biosimilar candidate to Stelara^{®}, or another product that contains human IgG1 kappa antibody and/or binds to the p40 subunit of human cytokines interleukin (IL)-12 and IL-23. In some embodiments, the drug delivery device may contain or be used with AmjevitaTM or AmgevitaTM (formerly ABP 501) (mab anti-TNF human IgG1), a biosimilar candidate to Humira^{®}, or another product that contains human mab anti-TNF human IgG1. In some embodiments, the drug delivery device may contain or be used with AMG 160, or another product that contains a half-life extended (HLE) anti-prostate-specific membrane antigen (PSMA) x anti-CD3 BiTE^{®} (bispecific T cell engager) construct. In some embodiments, the drug delivery device may contain or be used with AMG 119, or another product containing a delta-like ligand 3 (DLL3) CAR T (chimeric antigen receptor T cell) cellular therapy. In some embodiments, the drug delivery device may contain or be used with AMG 119, or another product containing a delta-like ligand 3 (DLL3) CAR T (chimeric antigen receptor T cell) cellular therapy. In some embodiments, the drug delivery device may contain or be used with AMG 133, or another product containing a gastric inhibitory polypeptide receptor (GIPR) antagonist and GLP-1R agonist. In some embodiments, the drug delivery device may contain or be used with AMG 171 or another product containing a Growth Differential Factor 15 (GDF15) analog. In some embodiments, the drug delivery device may contain or be used with AMG 176 or another product containing a small molecule inhibitor of myeloid cell leukemia 1 (MCL-1). In some embodiments, the drug delivery device may contain or be used with AMG 199 or another product containing a half-life extended (HLE) bispecific T cell engager construct (BiTE^{®}). In some embodiments, the drug delivery device may contain or be used with AMG 256 or another product containing an anti-PD-1 x IL21 mutein and/or an IL-21 receptor agonist designed to selectively turn on the Interleukin 21 (IL-21) pathway in programmed cell death-1 (PD-1) positive cells. In some embodiments, the drug delivery device may contain or be used with AMG 330 or another product containing an anti-CD33 x anti-CD3 BiTE^{®} (bispecific T cell engager) construct. In some embodiments, the drug delivery device may contain or be used with AMG 404 or another product containing a human anti-programmed cell death-1(PD-1) monoclonal antibody being investigated as a treatment for patients with solid tumors. In some embodiments, the drug delivery device may contain or be used with AMG 427 or another product containing a half-life extended (HLE) anti-fms-like tyrosine kinase 3 (FLT3) x anti-CD3 BiTE^{®} (bispecific T cell engager) construct. In some embodiments, the drug delivery device may contain or be used with AMG 430 or another product containing an anti-Jagged-1 monoclonal antibody. In some embodiments, the drug delivery device may contain or be used with AMG 506 or another product containing a multi-specific FAP x 4-1BB-targeting DARPin^{®} biologic under investigation as a treatment for solid tumors. In some embodiments, the drug delivery device may contain or be used with AMG 509 or another product containing a bivalent T-cell engager and is designed using XmAb^{®} 2+1 technology. In some embodiments, the drug delivery device may contain or be used with AMG 562 or another product containing a half-life extended (HLE) CD19 x CD3 BiTE^{®} (bispecific T cell engager) construct. In some embodiments, the drug delivery device may contain or be used with Efavaleukin alfa (formerly AMG 592) or another product containing an IL-2 mutein Fc fusion protein. In some embodiments, the drug delivery device may contain or be used with AMG 596 or another product containing a CD3 x epidermal growth factor receptor vIII (EGFRvIII) BiTE^{®} (bispecific T cell engager) molecule. In some embodiments, the drug delivery device may contain or be used with AMG 673 or another product containing a half-life extended (HLE) anti-CD33 x anti-CD3 BiTE^{®} (bispecific T cell engager) construct. In some embodiments, the drug delivery device may contain or be used with AMG 701 or another product containing a half-life extended (HLE) anti-B-cell maturation antigen (BCMA) x anti-CD3 BiTE^{®} (bispecific T cell engager) construct. In some embodiments, the drug delivery device may contain or be used with AMG 757 or another product containing a half-life extended (HLE) anti- delta-like ligand 3 (DLL3) x anti-CD3 BiTE^{®} (bispecific T cell engager) construct. In some embodiments, the drug delivery device may contain or be used with AMG 910 or another product containing a half-life extended (HLE) epithelial cell tight junction protein claudin 18.2 x CD3 BiTE^{®} (bispecific T cell engager) construct.

Although the drug delivery devices, assemblies, components, subsystems and methods have been described in terms of exemplary embodiments, they are not limited thereto. The detailed description is to be construed as exemplary only and does not describe every possible embodiment of the present disclosure. Numerous alternative embodiments could be implemented, using either current technology or technology developed after the filing date of this patent that would still fall within the scope of the claims defining the invention.

Those skilled in the art will recognize that a wide variety of modifications, alterations, and combinations can be made with respect to the above described embodiments without departing from the scope of the invention as defined by the appended claims.

## Claims

1. A drug delivery device (10) comprising:
a housing (12) having a proximal end (12a), a distal end (12b), and a longitudinal axis extending between the proximal end (12a) and the distal end (12b) thereof;
an injection assembly (15) at least partially disposed within the housing (12) at or near the proximal end (12a) thereof, the injection assembly (15) including a needle or a cannula, a drug storage container (23), and a container holder adapted to at least partially surround the drug storage container (23), the container holder including an arm (45);
a drive assembly (30) at least partially disposed within the housing (12) and operably coupled with the injection assembly (15), the drive assembly (30) including a trigger ring (54) being movable between an initial position and a releasing position to deliver a medicament via the injection assembly (15) and a nut (62) at least partially disposed around a periphery of the trigger ring (54), the nut (62) having at least one arm (63) formed on a portion thereof and adapted to engage a portion of the trigger ring (54) to prevent the trigger ring (54) from moving to the releasing position; and
a shield (32) slidably coupled with the housing (12) and operably coupled with the drive assembly (30), the shield (32) being positionable in an extended position in which at least a proximal end of the shield (32) extends a distance beyond the proximal end (12a) of the housing (12) and a retracted position in which the proximal end (12a) of the shield (32) is retracted, fully or partially, into the housing (12), wherein upon moving the shield (32) to the retracted position, a portion of the shield (32) urges the trigger ring (54) to the releasing position to deliver the medicament via the injection assembly (15);
wherein the container holder, the nut (62), and the trigger ring (54) cooperate to form a retention mechanism to prevent the trigger ring (54) from activating to deliver the medicament via the injection assembly (15) during unintentional movement of the housing (12) by engagement of the arm (45) of the container holder with the arm (63) of the nut (62).

2. The drug delivery device (10) of claim 1, wherein the retention mechanism includes:
an arm opening (55) formed on the trigger ring (54) adapted to receive at least a portion of the at least one arm (63) of the nut (62); and
wherein during unintended movement of the device (10), the container arm (45) is adapted to engage the at least one arm (63) of the nut (62) such that at least a portion of the at least one arm (63) of the nut (62) enters the arm opening (55) of the trigger ring (54) to prevent the trigger ring (54) from moving to the releasing position.

3. The drug delivery device (10) of claim 2, wherein the at least one arm (63) of the nut (62) is movable between an initial state and an engaged state, wherein in the engaged state, at least a portion of the at least one arm (63) of the nut (62) moves in a radially inward direction towards the longitudinal axis.

4. The drug delivery device (10) of claim 3, wherein during unintended movement of the device (10), the container arm (45) slidably engages the at least one arm (63) of the nut (62) to urge the at least one arm (63) of the nut (62) towards the longitudinal axis and into the arm opening (55) of the trigger ring (54).

5. The drug delivery device (10) of claim 3 or 4, wherein the at least one arm (63) of the nut (62) is biased towards the initial state.

6. The drug delivery device (10) of claim 5, wherein when the at least one arm (63) of the nut (62) is in the initial state, the at least one arm (63) of the nut (62) is removed from the arm opening (55) of the trigger ring (54) such that the trigger ring (54) may be moved to the releasing position.

7. The drug delivery device (10) of any one of claims 1 to 6, wherein the nut (62) is adapted to guide movement of the drive assembly (30) during medicament delivery.

8. The drug delivery device (10) of any one of claims 1 to 7, wherein the shield (32) includes an activator portion (34) adapted to engage the trigger ring (54).

9. The drug delivery device (10) of any one of claims 1 to 8, wherein movement of the shield (32) to the retracted position urges the trigger ring (54) to the releasing position.

10. The drug delivery device (10) of claim 9, wherein the shield (32) includes an activator portion (34) adapted to engage the trigger ring (54).

## Patentansprüche

1. Arzneimittelabgabevorrichtung (10), umfassend:
ein Gehäuse (12) mit einem proximalen Ende (12a), einem distalen Ende (12b) und einer Längsachse, die sich zwischen dem proximalen Ende (12a) und dem distalen Ende (12b) davon erstreckt;
eine Injektionsbaugruppe (15), die wenigstens teilweise innerhalb des Gehäuses (12) an oder nahe dem proximalen Ende (12a) davon angeordnet ist, wobei die Injektionsbaugruppe (15) eine Nadel oder eine Kanüle, einen Arzneimittelaufbewahrungsbehälter (23) und einen Behälterhalter beinhaltet, der angepasst ist, um den Arzneimittelaufbewahrungsbehälter (23) wenigstens teilweise zu umgeben, wobei der Behälterhalter einen Arm (45) beinhaltet;
eine Antriebsbaugruppe (30), die wenigstens teilweise innerhalb des Gehäuses (12) angeordnet und betriebsfähig mit der Injektionsbaugruppe (15) gekoppelt ist, wobei die Antriebsbaugruppe (30) einen Auslöserring (54), der zwischen einer Ausgangsposition und einer Freigabeposition beweglich ist, um ein Medikament über die Injektionsbaugruppe (15) abzugeben, und eine Mutter (62) beinhaltet, die wenigstens teilweise um einen Umfang des Auslöserrings (54) angeordnet ist, wobei die Mutter (62) wenigstens einen Arm (63) aufweist, der an einem Abschnitt davon ausgebildet und angepasst ist, um in einen Abschnitt des Auslöserrings (54) einzugreifen, um zu verhindern, dass sich der Auslöserring (54) in die Freigabeposition bewegt; und
eine Schutzvorrichtung (32), die verschiebbar mit dem Gehäuse (12) gekoppelt ist und betriebsfähig mit der Antriebsbaugruppe (30) gekoppelt ist, wobei die Schutzvorrichtung (32) in eine ausgefahrene Position, in der sich wenigstens ein proximales Ende der Schutzvorrichtung (32) um eine bestimmte Strecke über das proximale Ende (12a) des Gehäuses (12) hinaus erstreckt, und in eine eingefahrene Position positioniert werden kann, in der das proximale Ende (12a) der Schutzvorrichtung (32) ganz oder teilweise in das Gehäuse (12) eingefahren ist, wobei bei dem Bewegen der Schutzvorrichtung (32) in die eingefahrene Position ein Abschnitt der Schutzvorrichtung (32) den Auslöserring (54) in die Freigabeposition drängt, um das Medikament über die Injektionsbaugruppe (15) abzugeben;
wobei der Behälterhalter, die Mutter (62) und der Auslöserring (54) zusammenwirken, um einen Rückhaltemechanismus auszubilden, um zu verhindern, dass der Auslöserring (54) aktiviert wird, um das Medikament über die Injektionsbaugruppe (15) abzugeben, wenn das Gehäuse (12) unbeabsichtigt bewegt wird, indem der Arm (45) des Behälterhalters mit dem Arm (63) der Mutter (62) in Eingriff kommt.

2. Arzneimittelabgabevorrichtung (10) nach Anspruch 1, wobei der Haltemechanismus beinhaltet:
eine an dem Auslöserring (54) ausgebildete Armöffnung (55), die angepasst ist, um wenigstens einen Abschnitt des wenigstens einen Arms (63) der Mutter (62) aufzunehmen; und
wobei der Behälterarm (45) bei einer unbeabsichtigten Bewegung der Vorrichtung (10) angepasst ist, um in den wenigstens einen Arm (63) der Mutter (62) einzugreifen, sodass wenigstens ein Abschnitt des wenigstens einen Arms (63) der Mutter (62) in die Armöffnung (55) des Auslöserrings (54) eintritt, um zu verhindern, dass sich der Auslöserring (54) in die Freigabeposition bewegt.

3. Arzneimittelabgabevorrichtung (10) nach Anspruch 2, wobei der wenigstens eine Arm (63) der Mutter (62) zwischen einem Ausgangszustand und einem Eingriffszustand beweglich ist, wobei sich in dem Eingriffszustand wenigstens ein Abschnitt des wenigstens einen Arms (63) der Mutter (62) in einer radial nach innen gerichteten Richtung zu der Längsachse hin bewegt.

4. Arzneimittelabgabevorrichtung (10) nach Anspruch 3, wobei bei einer unbeabsichtigten Bewegung der Vorrichtung (10) der Behälterarm (45) gleitend in den wenigstens einen Arm (63) der Mutter (62) eingreift, um den wenigstens einen Arm (63) der Mutter (62) in Richtung der Längsachse und in die Armöffnung (55) des Auslöserrings (54) zu drängen.

5. Arzneimittelabgabevorrichtung (10) nach Anspruch 3 oder 4, wobei der wenigstens eine Arm (63) der Mutter (62) in Richtung des Ausgangszustands vorgespannt ist.

6. Arzneimittelabgabevorrichtung (10) nach Anspruch 5, wobei, wenn sich der wenigstens eine Arm (63) der Mutter (62) in dem Ausgangszustand befindet, der wenigstens eine Arm (63) der Mutter (62) aus der Armöffnung (55) des Auslöserrings (54) entfernt wird, sodass der Auslöserring (54) in die Freigabeposition bewegt werden kann.

7. Arzneimittelabgabevorrichtung (10) nach einem der Ansprüche 1 bis 6, wobei die Mutter (62) angepasst ist, um die Bewegung der Antriebsbaugruppe (30) bei der Medikamentenabgabe zu führen.

8. Arzneimittelabgabevorrichtung (10) nach einem der Ansprüche 1 bis 7, wobei die Schutzvorrichtung (32) einen Aktivatorabschnitt (34) beinhaltet, der angepasst ist, um in den Auslöserring (54) einzugreifen.

9. Arzneimittelabgabevorrichtung (10) nach einem der Ansprüche 1 bis 8, wobei die Bewegung der Schutzvorrichtung (32) in die eingefahrene Position den Auslösering (54) in die Freigabeposition drängt.

10. Arzneimittelabgabevorrichtung (10) nach Anspruch 9, wobei die Schutzvorrichtung (32) einen Aktivatorabschnitt (34) beinhaltet, der angepasst ist, um in den Auslöserring (54) einzugreifen.

## Revendications

1. Dispositif d'administration de médicament (10) comprenant :
un boîtier (12) présentant une extrémité proximale (12a), une extrémité distale (12b) et un axe longitudinal s'étendant entre l'extrémité proximale (12a) et l'extrémité distale (12b) de celui-ci,
un ensemble d'injection (15) disposé au moins partiellement à l'intérieur du boîtier (12), au niveau ou à proximité de l'extrémité proximale (12a) de celui-ci, l'ensemble d'injection (15) comprenant une aiguille ou une canule, un récipient de stockage de médicament (23) et un organe de maintien de récipient adapté à entourer au moins partiellement le récipient de stockage de médicament (23), l'organe de maintien de récipient comprenant un bras (45),
un ensemble d'actionnement (30) disposé au moins partiellement à l'intérieur du boîtier (12) et couplé de manière fonctionnelle à l'ensemble d'injection (15), l'ensemble d'actionnement (30) comprenant un anneau de déclenchement (54) mobile entre une position initiale et une position de libération permettant d'administrer un médicament par l'intermédiaire de l'ensemble d'injection (15), ainsi qu'un écrou (62) disposé au moins partiellement sur la périphérie de l'anneau de déclenchement (54), l'écrou (62) présentant au moins un bras (63) formé sur une portion de celui-ci et adapté à s'accoupler avec une portion de l'anneau de déclenchement (54) afin d'empêcher l'anneau de déclenchement (54) de passer en position de libération, et
un protège-embout (32) couplé de manière coulissante au boîtier (12) et couplé de manière fonctionnelle à l'ensemble d'actionnement (30), le protège-embout (32) pouvant prendre une position déployée dans laquelle au moins une extrémité proximale du protège-embout (32) s'étend sur une certaine distance au-delà de l'extrémité proximale (12a) du boîtier (12) et une position rétractée dans laquelle l'extrémité proximale (12a) du protège-embout (32) est rétractée, entièrement ou partiellement, à l'intérieur du boîtier (12) ; lorsque le protège-embout (32) passe en position rétractée, une portion du protège-embout (32) pousse l'anneau de déclenchement (54) vers la position de libération permettant d'administrer le médicament par l'intermédiaire de l'ensemble d'injection (15),
l'organe de maintien de récipient, l'écrou (62) et l'anneau de déclenchement (54) coopérant pour former un mécanisme de retenue permettant d'empêcher l'anneau de déclenchement (54) de s'activer pour administrer le médicament par l'intermédiaire de l'ensemble d'injection (15) lors d'un mouvement involontaire du boîtier (12), grâce à l'accouplement du bras (45) de l'organe de maintien de récipient avec le bras (63) de l'écrou (62).

2. Dispositif d'administration de médicament (10) selon la revendication 1, dans lequel le mécanisme de retenue comprend :
une ouverture de bras (55) formée sur l'anneau de déclenchement (54) et adaptée à recevoir au moins une portion de l'au moins un bras (63) de l'écrou (62), et
lors d'un mouvement involontaire du dispositif (10), le bras de récipient (45) est adapté à s'accoupler avec l'au moins un bras (63) de l'écrou (62) de sorte qu'au moins une portion de l'au moins un bras (63) de l'écrou (62) pénètre dans l'ouverture de bras (55) de l'anneau de déclenchement (54) afin d'empêcher l'anneau de déclenchement (54) de passer en position de libération.

3. Dispositif d'administration de médicament (10) selon la revendication 2, dans lequel l'au moins un bras (63) de l'écrou (62) est mobile entre un état initial et un état accouplé ; dans l'état accouplé, au moins une portion de l'au moins un bras (63) de l'écrou (62) se déplace, dans une direction radialement vers l'intérieur, vers l'axe longitudinal.

4. Dispositif d'administration de médicament (10) selon la revendication 3, dans lequel, lors d'un mouvement involontaire du dispositif (10), le bras de récipient (45) s'accouple de manière coulissante avec l'au moins un bras (63) de l'écrou (62) afin de pousser l'au moins un bras (63) de l'écrou (62) vers l'axe longitudinal et dans l'ouverture de bras (55) de l'anneau de déclenchement (54).

5. Dispositif d'administration de médicament (10) selon la revendication 3 ou 4, dans lequel l'au moins un bras (63) de l'écrou (62) est sollicité vers l'état initial.

6. Dispositif d'administration de médicament (10) selon la revendication 5, dans lequel, l'au moins un bras (63) de l'écrou (62) se trouvant dans l'état initial, l'au moins un bras (63) de l'écrou (62) est retiré de l'ouverture de bras (55) de l'anneau de déclenchement (54) de façon que l'anneau de déclenchement (54) puisse passer en position de libération.

7. Dispositif d'administration de médicament (10) selon l'une quelconque des revendications 1 à 6, dans lequel l'écrou (62) est adapté à guider le déplacement de l'ensemble d'actionnement (30) pendant l'administration de médicament.

8. Dispositif d'administration de médicament (10) selon l'une quelconque des revendications 1 à 7, dans lequel le protège-embout (32) comprend une portion d'activation (34) adaptée à s'accoupler avec l'anneau de déclenchement (54).

9. Dispositif d'administration de médicament (10) selon l'une quelconque des revendications 1 à 8, dans lequel le passage du protège-embout (32) en position rétractée pousse l'anneau de déclenchement (54) vers la position de libération.

10. Dispositif d'administration de médicament (10) selon la revendication 9, dans lequel le protège-embout (32) comprend une portion d'activation (34) adaptée à s'accoupler avec l'anneau de déclenchement (54).
